# EUROPÄISCHE PATENTSCHRIFT

(11) **EP 0 523 499 B1**
(45) Veröffentlichungstag und Bekanntmachung des Hinweises auf die Patenterteilung: **29.03.1995**
(21) Anmeldenummer: 92111427.8
(22) Anmeldetag: 06.07.1992
(51) Int. Cl.: C07D 333/20, C08B 37/16, A61K 31/38, A61K 31/715

(54) **Neue Acetohydroxamsäureverbindung, diese enthaltende Arzneimittel und Verfahren zur Herstellung dieser Verbindung und Arzneimittel**
Acetohydroxamic acid compound, medicaments containing it and method for the preparation of this compound and the medicaments
Composé d'acide acétohydroxamique, médicaments le contenant et procédé de préparation de ce composé et compositions pharmaceutiques

(30) Priorität: 17.07.1991 DE 4123613
(43) Veröffentlichungstag der Anmeldung: 20.01.1993
(73) Patentinhaber: Grünenthal GmbH, D-52078 Aachen (DE)
(72) Erfinder: Seipp, Ulrich, Dr., W-5100 Aachen (DE); Englberger, Werner, Dr., W-5190 Stolberg (DE); Haurand, Michael, Dr., W-5190 Stolberg (DE); Schneider, Johannes, Dr., W-5190 Stolberg (DE)

(56) Entgegenhaltungen:
- EP-A- 0 196 184
- EP-A- 0 412 939
- EP-A- 0 449 722
- WO-A-87/04152
- JOURNAL OF MEDICINAL CHEMISTRY. Bd. 31, Nr. 10, 1988, WASHINGTON US Seiten 1960 - 1964 J.B. SUMMERS ET AL. 'Structure-Activity Analysis of a Class of Orally Active Hydroxamic Acid Inhibitors of Leukotriene Biosynthesis'

## Beschreibung

Polyungesättigte höhere Fettsäuren, wie z. B. die Arachidonsäure dienen im Stoffwechsel des Menschen und von Säugetieren als Substrate für die enzymatisch katalysierte Bildung physiologisch bedeutsamer Eicosanoide wie z. B. Prostaglandine und Leukotriene, einer auch unter dem Namen "Slow reacting Substance of Anaphylaxis" (SRS-A) bekannten Substanzklasse. Dabei wird die Prostaglandinbildung durch die Cyclooxygenase (auch als "Prostaglandinsynthetase" bezeichnet), die Leukotrienbildung durch die 5-Lipoxygenase katalysiert.

Während die Prostaglandine eine Anzahl erwünschter Wirkungen im Organismus entfalten, ist von den Leukotrienen bzw. der SRS-A bekannt, daß sie für die Entstehung von allergischen Reaktionen bis zum anaphylaktischen Schock, Bronchokonstriktionen, Entzündungen, Asthma und einer Vielzahl weiterer unerwünschter Effekte verantwortlich sind. Es wird daher seit längerer Zeit angestrebt, über chemisch und metabolisch stabile Verbindungen verfügen zu können, die im Organismus die Prostaglandinbildung unbeeinflußt lassen, gleichzeitig aber möglichst selektiv bzw. spezifisch die 5-Lipoxygenase hemmen und so die Bildung der unerwünschten Leukotriene verhindern. Beispielsweise sind Acetohydroxamsäureverbindungen, die die 5-Lipoxygenase ausgeprägt, die Cyclooxygenase aber nur wenig hemmen, aus einer Arbeit von Tateson et al. in Brit. J. Pharmacol. (1988) 94, 528 - 539 bekannt. Auch die in EP 412 939 offenbarten Benzopyran- und Benzothiopyranderivate zeigen gegenüber 5-Lipoxygenase eine stärkere Hemmwirkung.

Acetohydroxamsäureverbindungen mit einer gegebenenfalls substituierten Phenylgruppe werden in WO 87/04152 und J. Med. Chem. 31, 1960 - 1964 (1988) beschrieben. Diese Verbindungen besitzen eine inhibierende Wirkung sowohl gegenüber 5-Lipoxygenase als auch gegenüber Cyclooxygenase.

Es wurde nunmehr gefunden, daß die neue Acetohydroxamsäureverbindung der Formel I 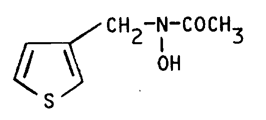
sowie deren Einschlußverbindungen (Komplexe) mit β-Cyclodextrin oder Hydroxypropyl-β-cyclodextrin sowohl bei parenteraler als auch bei oraler Verabreichung eine weitgehend selektive Hemmwirkung gegenüber der 5-Lipoxygenase besitzen und sich daher zum Einsatz als Therapeutika bzw. Prophylaktika bei Erkrankungen eignen, für die die unter der enzymatischen Wirkung der 5-Lipoxygenase entstehenden Leukotriene als Mediatoren fungieren. Solche Erkrankungen sind beispielsweise Asthma, rheumatoide Arthritis, Psoriasis, allergische Rhinitis, Endotoxin-Schocks, anaphylaktische Schocks, ischämischer Herzinfarkt sowie Störungen der coronaren und/oder cerebralen Gefäße.

Gegenstand der Erfindung sind dementsprechend die Acetohydroxamsäureverbindung der Formel I 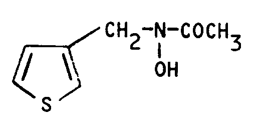
und deren Einschlußverbindungen mit β-Cyclodextrin oder Hydroxypropyl-β-cyclodextrin.

Weiterer Erfindungsgegenstand ist ein Verfahren zur Herstellung der Acetohydroxamsäureverbindung der Formel I und deren Einschlußverbindungen mit β-Cyclodextrin oder Hydroxypropyl-β-cyclodextrin, welches dadurch gekennzeichnet ist, daß man 3-Thiophencarboxaldehyd mit Hydroxylamin oder einem von dessen Salzen in Gegenwart von Basen zum Oxim umsetzt, dieses mittels borhaltigen Reduktionsmitteln in Gegenwart von Säuren zu N-(Thien-3-yl)methyl-hydroxylamin reduziert, in dieses den Acetylrest einführt und die so erhaltene Acetohydroxamsäureverbindung der Formel I gegebenenfalls anschließend durch Lösen in einer wäßrigen Lösung von β-Cyclodextrin oder Hydroxypropyl-β-cyclodextrin und Gefriertrocknung dieser Lösung in die entsprechende Einschlußverbindung überführt.

Die Umsetzung von 3-Thiophencarboxaldehyd zu dem entsprechenden Oxim erfolgt in an sich bekannter Weise, z. B. in alkoholischer oder wäßrig-alkoholischer Lösung in Gegenwart von Basen, z. B. Pyridin, Kaliumcarbonat oder Natriumacetat bei Temperaturen von 20 - 60° C.

Die Reduktion des Oxims wird vorzugsweise mit Borhydriden, insbesondere Natriumcyanoborhydrid, in Essigsäure oder ethanolischer Salzsäure bei Temperaturen zwischen 20° C und 60° C oder in alkoholischer Lösung mit Boran-Amin-Komplexen, z. B. dem Boran-Trimethylamin-Komplex oder dem Boran-Pyridin-Komplex, oder mit dem Boran-Tetrahydrofuran-Komplex, jeweils in Gegenwart von z. B. 6n-Salzsäure bei Temperaturen zwischen 0° C und 50° C durchgeführt (vgl. u. a. J. B. Summers et al., J. Med. Chem. 31, 1960 (1988)).

Durch Einführung des Acetylrestes erhält man dann aus N-(Thien-3-yl)methyl-hydroxylamin die gewünschte Acetohydroxamsäureverbindung N-Hydroxy-N-(thien-3-yl)methyl-acetamid. Dazu wird N-(Thien-3-yl)methyl-hydroxylamin gegebenenfalls ohne vorherige Isolierung mit einem Acetylierungsmittel, vorzugsweise Acetanhydrid oder Acetylchlorid, in Gegenwart säurebindender Stoffe wie Pyridin oder Chinolin umgesetzt, wobei die Verbindung der Formel II 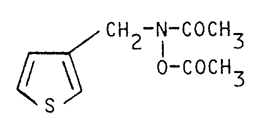
erhalten wird, aus der dann durch Zugabe von Basen in Methanol oder Ethanol als Lösungsmittel bei Temperaturen zwischen etwa 20° C und 60° C die O-Acetylgruppe unter Bildung der gewünschten Acetohydroxamsäureverbindung der Formel I abgespalten wird. Als Basen eignen sich z. B. Kalium-, Natrium- und/oder Lithiumhydroxid, Natrium- und/oder Kaliumcarbonat und/oder Natriumhydrogencarbonat, die gegebenenfalls in Form wäßriger 0,1 - 1 normaler Lösungen einer alkoholischen Lösung der Verbindung der Formel II zugesetzt werden.

Zur Herstellung von Einschlußverbindungen der Acetohydroxamsäureverbindung der Formel I mit β-Cyclodextrin oder Hydroxypropyl-β-cyclodextrin sättigt man wäßrige, gegebenenfalls natriumchloridhaltige Lösungen der genannten Cyclodextrine mit N-Hydroxy-N-(thien-3-yl)methyl-acetamid, filtriert und lyophilisiert dann das Filtrat. Die so erhaltenen Einschlußverbindungen zeichnen sich durch sehr gute Wasserlöslichkeit bei guter Bioverfügbarkeit des darin vorliegenden Wirkstoffes aus.

Wie bereits einleitend ausgeführt, besitzt die Acetohydroxamsäureverbindung der Formel I eine weitgehend selektive Hemmwirkung gegenüber der 5-Lipoxygenase, was u. a. durch in-vitro-Experimente ermittelt wurde.

Zur Ermittlung der 5-Lipoxygenasehemmung wurden Aliquote von frisch gewonnenem, heparinisiertem Humanblut entweder mit N-Hydroxy-N-(thien-3-yl)methyl-acetamid in unterschiedlichen Konzentrationen oder mit Lösungsmittel versetzt und bei 37° C im Wasserbad inkubiert. Nach 5 Minuten wurde Calcium-Ionophor A 23187 in einer Endkonzentration von 15 »g/ml Blut zugegeben und weitere 30 Minuten bei 37° C inkubiert. Zur Gewinnung des zellfreien Plasmas wurden anschließend die Inkubationsansätze zentrifugiert. Im zellfreien Plasma wurde der Gehalt an immunreaktivem Leukotrien B₄ ("iLTB₄") per Radio-Immun-Assay "RIA" (³H-LTB₄-RIA, Fa Amersham) bestimmt. Der iLTB₄-Gehalt jeder Probe wurde anhand einer mit verdünntem Humanplasma erstellten Standardkurve von LTB₄-Standardkonzentrationen rechnerisch ermittelt und als ng iLTB₄ pro ml Humanplasma berechnet. Gegenüber den Ansätzen mit Lösungsmittel wurde die prozentuale Hemmung in Gegenwart unterschiedlicher Konzentrationen der Acetohydroxamsäureverbindung der Formel I für die jeweilige Konzentration berechnet. Der IC₅₀-Wert, d. h. die Konzentration, die eine 50 %ige Hemmung der 5-Lipoxygenase bewirkt, wurde für N-Hydroxy-N-(thien-3-yl)methyl-acetamid mittels linearer Regressionsgerade mit 0,3 »molar ermittelt.

An zellfreier 5-Lipoxygenase aus basophilen leukämischen Leukozyten der Ratte (RBL-1-Zellen) wurde untersucht, wie schnell und bis zu welchem Ausmaß N-Hydroxy-N-(thien-3-yl)methyl-acetamid aus der nach Beispiel 3 hergestellten Einschlußverbindung freigesetzt wird. Als Meßparameter wurde die 5-Lipoxygenaseaktivität im 10.000 x g Überstand von RBL-1-Zellhomogenaten polarographisch mit Sauerstoffelektroden gemessen. Nach 5-minütiger Vorinkubation des 10.000 x g Überstandes mit 75 »M Arachidonsäure, 4 mM Adenosintriphosphat, 4 mM Glutathion und entweder N-Hydroxy-N-(thien-3-yl)methyl-acetamid oder Lösungsmittel bei 35° C wurde die Lipoxygenasereaktion durch Zugabe von CaCl₂ gestartet und simultan aufgezeichnet. In die Auswertung wurde sowohl die Dauer der Lag-Phase als auch die Anfangsgeschwindigkeit der Reaktion nach der Lag-Phase einbezogen. Dabei zeigte sich, daß die durch die nach Beispiel 3 hergestellte Einschlußverbindung bewirkten Effekte sowohl bezüglich der Verlängerung der Lag-Phase als auch bezüglich der Hemmung der 5-Lipoxygenasereaktion mit den durch die in freier Form eingesetzte Acetohydroxamsäureverbindung der Formel I bedingten Effekten vergleichbar sind. Weiterhin zeigten Untersuchungen mit verschiedenen Vorinkubationszeiten, daß die Acetohydroxamsäureverbindung ohne relevante zeitliche Verzögerung aus der Einschlußverbindung freigesetzt wird. Die Acetohydroxamsäureverbindung liegt also auch bei Verwendung in Form der nach Beispiel 3 hergestellten Einschlußverbindung im biologischen Testsystem zumindest zu einem bedeutenden Teil frei verfügbar vor.

Der Einfluß der Acetohydroxamsäureverbindung der Formel I auf die Cyclooxygenaseaktivität wurde mit Hilfe von Schafsamenblasenmikrosomen, suspendiert in Puffer (Kaliumphosphat 50 mM, pH 7,0), durch Inkubation entweder mit N-Hydroxy-N-(thien-3-yl)methyl-acetamid oder mit Lösungsmittel und radioaktiv markierter Arachidonsäure untersucht. Die IC₅₀-Werte für die Hemmung der Cyclooxygenase wurden mittels Dünnschichtchromatographie und TLC-Linear-Analyzer bestimmt. Dabei ergab sich, daß die IC₅₀-Werte für die Cyclooxygenasehemmung wesentlich höher als die Werte für die 5-Lipoxygenasehemmung liegen, d. h. daß die Acetohydroxamsäureverbindung der Formel I weitgehend selektiv 5-Lipoxygenaseaktivität hemmt.

Diese vorteilhaften Eigenschaften der neuen Acetohydroxamsäureverbindung waren aufgrund des Standes der Technik nicht vorhersehbar. Beispielsweise wird in der veröffentlichten europäischen Patentanmeldung 0 351 214 ein Acetohydroxamsäurederivat mit Hemmwirkung sowohl gegenüber Lipoxygenase als auch gegenüber Cyclooxygenase beschrieben.

Die europäische Patentanmeldung 0 196 184 bezieht sich u. a. auf Acylhydroxamsäurederivate, für die ebenfalls die Hemmwirkung gegenüber Lipoxygenase und/oder Cyclooxygenase (insbesondere auch allein gegen letzteres Enzym - vergleiche Spalte 13, Zeilen 26 bis 29) herausgestellt wird. Eine Vorhersage, ob und gegen welches Enzym die erfindungsgemäße neue Acetohydroxamsäureverbindung eine weitgehend selektive Hemmwirkung entfalten könnte, war daher nicht möglich.

Zur Überprüfung der oralen in-vivo-Verfügbarkeit als 5-Lipoxygenasehemmer wurde die Acetohydroxamsäureverbindung der Formel I männlichen Ratten (Stamm Wistar) in einer Dosis von 21,5 mg/kg peroral appliziert und die nach Resorption der Verbindung resultierende 5-Lipoxygenasehemmung ex vivo im Vollblut bestimmt (Tateson et al., Brit. J. Pharmacol. 94, 528 (1988). Jeweils 1 Stunde nach Applikation wurde den Tieren nach letaler CO₂-Narkose unter Zusatz von Heparin als Antikoagulans Vollblut entnommen. Aliquote des Vollblutes wurden im Wasserbad bei 37° C 30 Minuten mit dem Calciumionophor A 23187 in einer Endkonzentration von 15 »g/ml inkubiert. Anschließend wurden die Inkubationsansätze zentrifugiert und in aliquoten Teilen des zellfreien Plasmas der Gehalt an immunreaktivem LTB₄ ("iLTB₄") per Radio-Immun-Assay "RIA" (³H-LTB₄-RIA, Fa. Amersham) bestimmt. Der iLTB₄-Gehalt jeder Probe wurde anhand einer mit verdünntem Rattenplasma erstellten Standardkurve von LTB₄-Standardkonzentrationen rechnerisch ermittelt und als ng iLTB₄/ml Rattenplasma berechnet. Parallel zu den mit N-Hydroxy-N(thien-3-yl)methyl-acetamid behandelten Tieren wurden jeweils mit Vehikellösung peroral behandelte Tiere als Kontrollgruppe mitgeführt. Der Mittelwert aus den iLTB₄-Gehalten der Rattenplasmen dieser Kontrollgruppe diente als 100 %-Wert. Die jeweilige prozentuale Hemmung der ex vivo LTB₄-Synthese nach oraler Applikation der Acetohydroxamsäureverbindung wurde wie folgt berechnet: 100 minus dem 100-fachen des Quotienten aus dem Mittelwert der iLTB₄-Gehalte der mit der Acetohydroxamsäureverbindung behandelten Gruppe und dem Mittelwert der iLTB₄-Gehalte der Kontrollgruppe (immer in ng iLTB₄/ml). Für die Acetohydroxamsäureverbindung ergab sich eine Hemmung von 95 %, das bedeutet eine sehr gute orale Verfügbarkeit (als Dosis mit halbmaximaler Hemmwirkung nach oraler Applikation wurde 5,6 mg/kg errechnet).

An narkotisierten, curarisierten und passiv beatmeten Meerschweinchen wurde die antiasthmatische Wirkung der Acetohydroxamsäureverbindung der Formel I untersucht. Zur Auslösung einer asthmatischen Reaktion wurden die Tiere passiv mit Antiovalbumin Serum (0,3 ml intraperitoneal) sensibilisiert. Nach 48 Stunden wurde die asthmatische Reaktion durch intravenöse Gabe von 0,2 mg/kg Ovalbumin ausgelöst. Die sofort auftretende Bronchokonstriktion wurde nach der Konzett-Rössler-Methode (Naunyn Schmiedeberg's Arch. Exp. Path. Pharma. Vol. 195 (1940), Seiten 71 - 74) anhand des intra-trachealen Druckanstiegs über einen Zeitraum von 15 Minuten gemessen. Effekte, die durch Histamin, Serotonin und sympathische Gegensteuerung ausgelöst werden, wurden durch intravenöse Vorbehandlung der Tiere mit 2,15 mg/kg Mepyramin, 46,4 mg/kg Propanolol, 4,64 mg/kg Atropin und 1 mg/kg Methysergid (5 Minuten vor Auslösung der Bronchokonstriktion durch Gabe von Ovalbumin) ausgeschaltet. N-Hydroxy-N-(thien-3-yl)methyl-acetamid wurde bei oraler Applikation 60 Minuten vor der Ovalbumin-Verabreichung, bei intravenöser Applikation 30 Minuten und bei lokaler Applikation (intratracheale Instillation) 2 Minuten vorher gegeben. Die Tabelle zeigt die Bronchokonstriktionshemmung gegenüber einer nur mit Vehikellösung vorbehandelten Kontrollgruppe.

| Acetohydroxamsäureverbindung gemäß Beispiel | Dosis [mg/kg] | Applikationsweg | Hemmung der Bronchokonstriktion |
|---|---|---|---|
| 1 | 21,5 | oral | 42 % |
| 3 | 21,5*⁾ | intravenös | 49 % |
| 3 | 10,0*⁾ | lokal | 56 % |

| | | | |
|---|---|---|---|
| *) bezogen auf den Gehalt an eingeschlossenem N-Hydroxy-N-(thien-3-yl)methyl-acetamid | | | |

An den Ergebnissen aus dieser Tabelle ist bemerkenswert, daß die Einschlußverbindung von Beispiel 3 bei der lokalen (intratrachealen) Applikation in ca. der halben Dosis eine stärkere Hemmwirkung als bei intravenöser Verabreichung auslöste. Aus dem Befund, daß N-Hydroxy-N-(thien-3-yl)methyl-acetamid bei oraler Applikation von 21,5 mg/kg gleich gut wirksam war wie dieselbe Verbindung in gleicher Menge in Form der Einschlußverbindung aus Beispiel 3 bei intravenöser Verabreichung ergibt sich eine hohe Resorptionsquote für N-Hydroxy-N-(thien-3-yl)methyl-acetamid.

Die antianaphylaktische Wirkung von N-Hydroxy-N-(thien-3-yl)methyl-acetamid wurde auch in einem in-vitro-Modell nachgewiesen. Dazu wurden isolierte, spiralig präparierte Trachealstreifen von aktiv gegen Ovalbumin (i.p.-Applikation 3 Wochen vor Organentnahme, 10 »g zusammen mit 20 mg Aluminiumhydroxyd als Adjuvans) sensibilisierten Meerschweinchen in mit Carbogen (einem Gemisch aus 95 % Sauerstoff und 5 % Kohlendioxid) begaster Nährlösung gefüllte und auf 37° C temperierte Organbäder gebracht und mit isometrischen Kraftaufnehmern verbunden. Nach Vorinkubation mit Mepyramin (Histamin-Ausschaltung) und Indomethacin (Hemmung der Cyclooxygenase, dadurch Verstärkung der anaphylaktischen Reaktion) wurde das Antigen (Ovalbumin) in kumulativ ansteigenden Konzentrationen dem Organbad zugegeben. Die dadurch ausgelösten Kontraktionen wurden durch N-Hydroxy-N-(thien-3-yl)methyl-acetamid, das vor Auslösung der anaphylaktischen Kontraktionen in das Organbad gegeben wurde, konzentrationsabhängig gehemmt. In der Tabelle sind die IC₅₀-Werte, d. h. die Konzentrationen, die eine 50 %ige Hemmung der anaphylaktischen Kontraktion an isolierten Trachealstreifen des Meerschweinchens bewirken, angegeben.

| Acetohydroxamsäureverbindung gemäß Beispiel | IC₅₀ [»molar] |
|---|---|
| 1 | 10,2 |
| 3*⁾ | 18,7 |

| | |
|---|---|
| *) bezogen auf den Gehalt an eingeschlossenem N-Hydroxy-N-(thien-3-yl)methyl-acetamid | |

Die erfindungsgemäße Verbindung der Formel I besitzt aufgrund ihrer weitgehend selektiven Hemmung der 5-Lipoxygenase und damit der Bildung von Arachidonsäuremetaboliten, wie 5-Hydroperoxyeicosatetraensäure (5-HPETE), 5-Hydroxyeicosatetraensäure (5-HETE) bzw. SRS-A zahlreiche physiologisch wertvolle Eigenschaften, beispielsweise antianaphylaktische, antiasthmatische, antiallergische sowie antiphlogistische, blutdrucksenkende und durchblutungsfördernde (coronarer und cerebraler Kreislauf) Wirkungen, eine verminderte Leukozytenaggregation sowie eine geringere Bildung von Leukozytenthromben. Da die erfindungsgemäße Verbindung der Formel I chemisch und metabolisch für eine therapeutische Anwendung stabil und lagerfähig ist, eignet sie sich zum Einsatz als Arzneimittel, z. B. als Antiallergikum, Antianaphylaktikum, Antiphlogistikum, Antiasthmatikum, Antihypertensivum, antithrombotisches Mittel, als Mittel zur Prophylaxe oder Therapie von ischämischem Herzinfarkt, gegen Störungen der coronaren und/oder cerebralen Gefäße oder gegen Morbus Crohn.

N-Hydroxy-N-(thien-3-yl)methyl-acetamid besitzt nur eine geringe Toxizität, die sich erst bei weit höheren als den therapeutisch oder prophylaktisch anzuwendenden Dosierungen bemerkbar macht. Sie kann daher in geeigneten pharma zeutischen Zubereitungen an Mensch oder Tier verabreicht werden.

Die Erfindung betrifft dementsprechend auch Arzneimittel, die als Wirkstoff N-Hydroxy-N-(thien-3-yl)methyl-acetamid gegebenenfalls in Form einer Einschlußverbindung mit β-Cyclodextrin oder Hydroxypropyl-β-cyclodextrin enthalten. Die an den Patienten zu verabreichende Wirkstoffmenge variiert in Abhängigkeit z. B. vom Gewicht des Patienten, vom Applikationsweg, der Indikation und dem Schweregrad der Erkrankung. Unter Berücksichtigung dieser Faktoren beträgt der Wirkstoffgehalt pro Einzeldosis im allgemeinen etwa 0,01 bis 1000 mg, und zwar bei Zubereitungsformen für die parenterale Applikation 0,1 bis 1000 mg, bei Zubereitungsformen für die orale oder rektale Applikation 0,1 bis 1000 mg und bei Zubereitungsformen für die topische oder inhalative Applikation 0,01 bis 100 mg.

Arzneimittel zur parenteralen Applikation können sowohl Lösungen als auch Suspensionen darstellen, es kommen aber auch leicht rekonstituierbare Trockenzubereitungen in Betracht.

Gut geeignete Applikationsformen sind auch Sprays zur intranasalen oder oralen Applikation bzw. zur Verabreichung der Substanzen über die Bronchien.

Für viele prophylaktische oder therapeutische Anwendungen kommen zweckmäßig auch oral anwendbare Zubereitungsformen der Acetohydroxamsäureverbindung der Formel I oder deren Einschlußverbindungen mit Cyclodextrin oder Hydroxypropyl-β-cyclodextrin, wie Tabletten, Dragees, Kapseln, Granulate, Tropfen und Säfte bzw. Sirupe, aber auch Suppositorien sowie perkutane Applikationszubereitungen, wie z. B. Wirkstoffe in einem Depot in gelöster Form oder gegebenenfalls unter Zusatz von die Hautpenetration fördernden Mitteln enthaltende Pflaster, in Betracht. Vorteilhaft werden diese oral, rektal oder perkutan anwendbaren Zubereitungsformen so hergestellt, daß daraus der Wirkstoff verzögert freigesetzt wird, um so über einen längeren Zeitraum (beispielsweise 24 Stunden) eine gleichförmige Versorgung des Patienten mit dem Wirkstoff zu gewährleisten.

Alle vorstehend erwähnten pharmazeutischen Zubereitungsformen sind an sich bekannt, und da die erfindungsgemäße Verbindung der Formel I chemisch stabil ist, wirft ihre Einarbeitung in diese Zubereitungsformen für den Fachmann keinerlei Probleme auf. Bei der erfindungsgemäßen Herstellung dieser Arzneimittel muß selbstverständlich mit der üblichen Sorgfalt bei Auswahl der Hilfsstoffe wie Trägermaterialien, Lösungsmittel, Verdünnungsmittel, Farbstoffe, Geschmackskorrigentien, Bindemittel, Tablettensprengmittel vorgegangen werden, und insbesondere bei der Herstellung von parenteral anzuwendenden Zubereitungsformen ist auf Sterilität und - wenn sie in flüssiger Form vorliegen - Isotonie zu achten.

### Beispiele

Alle Temperaturangaben sind unkorrigiert.

Die ¹H-NMR-Spektren wurden bei 300 MHz gemessen. Die chemische Verschiebung der spektroskopischen Resonanzdaten ist in ppm angegeben.

Als stationäre Phase für die Säulenchromatographie wurde Kieselgel 60 (0,040 - 0,063 mm) der Firma E. Merck, Darmstadt, eingesetzt.

Die dünnschichtchromatographischen Untersuchungen wurden mit "HPTLC Fertigplatten, Kieselgel 60 F 254" der Firma E. Merck, Darmstadt, durchgeführt.

Die Mischungsverhältnisse der Laufmittel für alle chromatographischen Untersuchungen sind in Volumen/Volumen angegeben.

### Beispiel 1

### Herstellung von N-Hydroxy-N-(thien-3-yl)methyl-acetamid

a) 3-Thiophencarboxaldehydoxim (syn/anti Gemisch)
   11,2 g 3-Thiophencarboxaldehyd, 7 g Hydroxylaminhydrochlorid und 5,5 g Natriumcarbonat wurden in 200 ml Ethanol/Wasser (1 : 1) 17 Stunden bei Raumtemperatur gerührt. Anschließend wurde das Reaktionsgemisch zu 500 ml gesättigter Natriumchloridlösung gegossen und dreimal mit je 250 ml Essigsäureethylester ausgeschüttelt. Die vereinigten organischen Phasen wurden mit gesättigter Natriumchloridlösung gewaschen, über Magnesiumsulfat getrocknet, filtriert und am Rotationsverdampfer bei 40° C im Vakuum eingedampft. Es wurden 14,19 g 3-Thiophencarboxaldehydoxim erhalten.
b) N-(Thien-3-yl)methyl-hydroxylamin
   11,45 g des nach Beispiel 1a erhaltenen Oxims wurden in 450 ml Eisessig gegeben, dann bei Raumtemperatur mit 9 g Natriumcyanoborhydrid versetzt und 2 Stunden bei Raumtemperatur gerührt.
c) N-Acetoxy-N-(thien-3-yl)methyl-acetamid
   Zu der nach Beispiel 1b erhaltenen Lösung von N-(Thien-3-yl)methyl-hydroxylamin wurden bei Raumtemperatur 70,9 ml Acetanhydrid gegeben. Nach 17stündigem Rühren wurde bei 60° C im Vakuum eingedampft, der Rückstand nach Zugabe von 150 ml Essigsäureethylester zu 700 ml Wasser gegossen und das Gemisch mit festem Natriumhydrogencarbonat bis zum Ausbleiben der Kohlendioxidbildung versetzt. Die organische Phase wurde abgetrennt und die wäßrige Phase mit Essigsäureethylester gewaschen. Anschließend wurden die vereinigten organischen Phasen mit Magnesiumsulfat getrocknet und bei 40° C im Vakuum eingedampft. Nach chromatographischer Reinigung mit Hexan/Isopropanol/Eisessig (4,5 : 0,5 : 0,05) wurden 13,27 g N-Acetoxy-N-(thien-3-yl)methyl-acetamid in Form eines gelblichen Öls erhalten.
d) N-Hydroxy-N-(thien-3-yl)methyl-acetamid
   Eine Lösung von 3,68 g der nach Beispiel 1c erhaltenen Bisacetylverbindung in 56 ml Methanol wurde bei Raumtemperatur mit 22,5 ml 1-molarer methanolischer Lithiumhydroxidlösung versetzt und 45 Minuten gerührt. Anschließend wurde das Reaktionsgemisch zu 150 ml gesättigter Natriumchloridlösung und 40 ml gesättigter Natriumhydrogencarbonatlösung gegeben und mit Essigsäureethylester ausgeschüttelt. Die vereinigten organischen Phasen wurden mit gesättigter Kochsalzlösung gewaschen, über Magnesiumsulfat getrocknet, filtriert und bei 40° C im Vakuum eingedampft. Nach chromatographischer Reinigung mit Hexan/Essigsäureethylester/Methanol (4 : 6 : 0,5) und Umkristallisation aus Essigsäureethylester wurden 1,8 g N-Hydroxy-N-(thien-3-yl)methyl-acetamid als farbloser Feststoff mit einem Schmelzpunkt von 92 - 93° C erhalten.
   - ¹H-NMR (DMSO-d₆):: 2,03 (s, 3H); 4,66 (s, 2H); 7,03 (m, 1H); 7,31 (d, 2Hz, 1H); 7,43 (m, 1H); 9,87 (s, 1H)

### Beispiel 2

### Herstellung einer Einschlußverbindung mit β-Cyclodextrin

Zu einer Lösung von 1,8 g β-Cyclodextrin in 100 ml Wasser wurden bei Raumtemperatur 1,36 g N-Hydroxy-N-(thien-3-yl)methyl-acetamid gegeben. Nach 17stündigem Rühren wurde filtriert und das Filtrat gefriergetrocknet, wobei 3,1 g eines farblosen Puffers erhalten wurden, welches 44 Gew.-% N-Hydroxy-N-(thien-3-yl)methyl-acetamid enthielt (Gehaltsbestimmung über Vergleich der UV-Extinktionen definierter Lösungen der Einschlußverbindung mit denen von N-Hydroxy-N-(thien-3-yl)methyl-acetamid).

### Beispiel 3

### Herstellung einer Einschlußverbindung mit Hydroxypropyl-β-cyclodextrin

Zu einer Lösung von 5 g Hydroxypropyl-β-cyclodextrin in 10 ml 0,9 gew.-%iger Natriumchloridlösung wurden 0,86 g N-Hydroxy-N-(thien-3-yl)methyl-acetamid gegeben und wie in Beispiel 2 beschrieben weitergearbeitet. Es wurden 5,3 g eines farblosen Pulvers erhalten, welches 14,3 Gew.-% N-Hydroxy-N-(thien-3-yl)methyl-acetamid enthielt (Gehaltsbestimmung in gleicher Weise wie in Beispiel 2).

Die nach den Beispielen 2 und 3 erhaltenen Einschlußverbindungen wiesen eine sehr gute Wasserlöslichkeit auf, woraus die bereits weiter oben aufgezeigte gute Bioverfügbarkeit resultiert. Da insbesondere das Hydroxypropyl-β-cyclodextrin gut verträglich ist, liegen in den damit erhaltenen Einschlußverbindungen von N-Hydroxy-N-(thien-3-yl)methyl-acetamid Produkte mit großen Anwendungsvorteilen für bestimmte Applikationsformen (oral zu verabreichende, parenteral anzuwendende Flüssigpräparate oder leicht in Lösung zu bringende Pulverzubereitungsformen) vor. In der nachfolgenden Tabelle sind einige Angaben zur Löslichkeit zusammengestellt:

| Lösungsmittel | Löslichkeit in mg/ml der Acetohydroxamsäureverbindung von Beispiel | | |
|---|---|---|---|
| | 1 | 2 | 3 |
| Phosphatpuffer pH 7,4/Ethanol 4 : 1 | 3 | | |
| Phosphatpuffer pH 7,4 | | 53 (a) | 560 (b) |
| Wasser/Ethanol 4 : 1 | 3 | | |
| Wasser | | 56 (c) | 360 (d) |

Die angegebenen Mengen der Einschlußverbindungen entsprachen folgenden Mengen an N-Hydroxy-N-(thien-3-yl)methyl-acetamid:

| | | | |
|---|---|---|---|
| a) | 22 mg/ml | b) | 82 mg/ml |
| c) | 23 mg/ml | d) | 53 mg/ml |

## Patentansprüche

1. Acetohydroxamsäureverbindung der Formel I 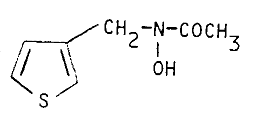 sowie deren Einschlußverbindungen mit β-Cyclodextrin oder Hydroxypropyl-β-cyclodextrin.

2. Verfahren zur Herstellung der Acetohydroxamsäureverbindung der Formel I 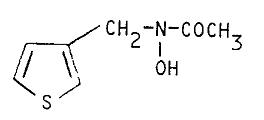 sowie deren Einschlußverbindungen mit β-Cyclodextrin oder Hydroxypropyl-β-cyclodextrin, dadurch gekennzeichnet, daß man 3-Thiophencarboxaldehyd mit Hydroxylamin oder einem von dessen Salzen in Gegenwart von Basen zum Oxim umsetzt, dieses mit borhaltigen Reduktionsmitteln in Gegenwart von Säuren zu N-(Thien-3-yl)methyl-hydroxylamin reduziert, in dieses den Acetylrest einführt und die so erhaltene Acetohydroxamsäureverbindung der Formel I gegebenenfalls anschließend durch Lösen in einer wäßrigen Lösung von β-Cyclodextrin oder Hydroxypropyl-β-cyclodextrin und Gefriertrocknung dieser Lösung in die entsprechende Einschlußverbindung überführt.

3. Verfahren nach Anspruch 2, dadurch gekennzeichnet, daß man N-(Thien-3-yl)methyl-hydroxylamin mit einem Acetylierungsmittel, vorzugsweise mit Acetanhydrid oder Acetylchlorid in Gegenwart säurebindender Stoffe zu der Verbindung der Formel II 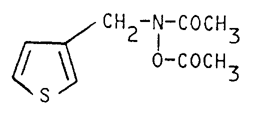 umsetzt und anschließend durch Zugabe von Basen die O-Acetylgruppe abspaltet.

4. Verfahren nach Anspruch 2, dadurch gekennzeichnet, daß man das Oxim mit einem Borhydrid, insbesondere Natriumcyanoborhydrid in Gegenwart von Säuren oder mit einem Boran-Amin-Komplex oder dem Boran-Tetrahydrofuran-Komplex in Gegenwart von Säuren zum N-(Thien-3-yl)methyl-hydroxylamin reduziert.

5. Arzneimittel, dadurch gekennzeichnet, daß es als Wirkstoff die Acetohydroxamsäureverbindung der Formel I als solche oder in Form einer Einschlußverbindung gemäß Anspruch 1 enthält und zur parenteralen, oralen, rektalen, topischen oder intranasalen Applikation geeignet ist.

6. Arzneimittel nach Anspruch 5, dadurch gekennzeichnet, daß es als Wirkstoff pro Einzeldosis 0,01 bis 1000 mg Acetohydroxamsäureverbindung der Formel I als solche oder in Form einer Einschlußverbindung gemäß Anspruch 1 enthält.

7. Arzneimittel nach einem oder beiden der Ansprüche 5 bis 6, dadurch gekennzeichnet, daß es zur parenteralen Applikation geeignet ist und als Wirkstoff pro Einzeldosis 0,1 bis 1000 mg Acetohydroxamsäureverbindung der Formel I als solche oder in Form einer Einschlußverbindung gemäß Anspruch 1 enthält.

8. Arzneimittel nach einem oder beiden der Ansprüche 5 bis 6, dadurch gekennzeichnet, daß es in Form von Tabletten, Dragees oder Kapseln, gegebenenfalls mit verzögerter Wirkstofffreigabe, vorliegt und als Wirkstoff pro Einzeldosis 0,1 bis 1000 mg Acetohydroxamsäureverbindung der Formel I als solche oder in Form einer Einschlußverbindung gemäß Anspruch 1 enthält.

9. Arzneimittel nach einem oder beiden der Ansprüche 5 bis 6 in Sprayform zur intranasalen oder oralen Applikation.

10. Verfahren zur Herstellung eines Arzneimittels nach einem oder mehreren der Ansprüche 5 bis 9, dadurch gekennzeichnet, daß man die Acetohydroxamsäureverbindung der Formel I als solche oder in Form einer Einschlußverbindung gemäß Anspruch 1 in bekannter Weise mit Trägermaterialien, Lösungsmitteln und/oder Verdünnungsmitteln, sowie gegebenenfalls Bindemitteln, Tablettensprengmitteln und anderen Hilfsstoffen verarbeitet und aus dem erhaltenen Gemisch eine Einzeldosierungsform herstellt.

## Claims

1. Acetohydroxamic acid compound of the formula I 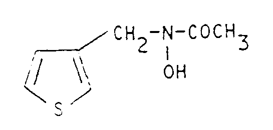 and the inclusion compounds thereof with β-cyclodextrin or hydroxypropyl-β-cyclodextrin.

2. Method for preparing the acetohydroxamic acid compound of the formula I 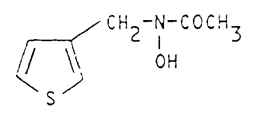 and the inclusion compounds thereof with β-cyclodextrin or hydroxypropyl-β-cyclodextrin, characterised in that 3-thiophenecarboxaldehyde is reacted with hydroxylamine or one of the salts thereof in the presence of bases to form the oxime, the latter is reduced by means of boron-containing reducing agents in the presence of acids to N-(thien-3-yl)methylhydroxylamine, into which the acetyl radical is introduced, and the acetohydroxamic acid compound of the formula I thus obtained is subsequently optionally converted into the corresponding inclusion compound by dissolving in an aqueous solution of β-cyclodextrin or hydroxypropyl-β-cyclodextrin and freeze-drying the said solution.

3. Method according to claim 2, characterised in that N-(thien-3-yl)methylhydroxylamine is converted using an acetylating agent, preferably using acetic anhydride or acetyl chloride in the presence of acid-binding substances, to the compound of the formula II 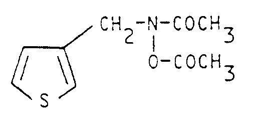 and the O-acetyl group is subsequently split off by the addition of bases.

4. Method according to claim 2, characterised in that the oxime is reduced to N-(thien-3-yl)methylhydroxylamine using a borohydride, in particular sodium cyanoborohydride in the presence of acids or using a borane-amine complex or the borane-tetrahydrofuran complex in the presence of acids.

5. Pharmaceutical, characterised in that it contains as the active substance the acetohydroxamic acid compound of the formula I as such or in the form of an inclusion compound according to claim 1 and is suitable for parenteral, oral, rectal, topical or intranasal application.

6. Pharmaceutical according to claim 5, characterised in that it contains as the active substance per single dose from 0.01 to 1000 mg of the acetohydroxamic acid compound of the formula I as such or in the form of an inclusion compound according to claim 1.

7. Pharmaceutical according to one or both of claims 5 and 6, characterised in that it is suitable for parenteral application and contains as the active substance per single dose from 0.1 to 1000 mg of the acetohydroxamic acid compound of the formula I as such or in the form of an inclusion compound according to claim 1.

8. Pharmaceutical according to one or both of claims 5 and 6, characterised in that it is in the form of tablets, dragées or capsules, optionally with delayed liberation of the active substance, and contains as the active substance per single dose from 0.1 to 1000 mg of the acetohydroxamic acid compound of the formula I as such or in the form of an inclusion compound according to claim 1.

9. Pharmaceutical according to one or both of claims 5 and 6 in spray form for intranasal or oral application.

10. Method for preparing a pharmaceutical according to one or more of claims 5 to 9, characterised in that the acetohydroxamic acid compound of the formula I, as such or in the form of an inclusion compound according to claim 1, is processed in a known manner with supporting materials, solvents and/or thinners as well as optionally binders, disintegrants and other auxiliary substances and a single dose form of preparation is prepared from the mixture obtained.

## Revendications

1. Composé de l'acide acéto-hydroxamique de formule I 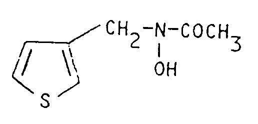 ainsi que ses composés d'inclusion avec la β-cyclodextrine ou l'hydroxypropyl-β-cyclodextrine.

2. Procédé de production du composé d'acide acéto-hydroxamique de formule I 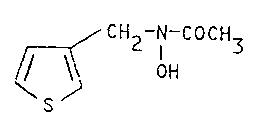 ainsi que ses composés d'inclusion avec la β-cyclodextrine ou l'hydroxypropyl-β-cyclodextrine, caractérisé en ce qu'on fait réagir du 3-thiophène-carboxaldéhyde avec l'hydroxylamine ou l'un de ses sels en présence de bases pour former une oxime, on réduit cette dernière avec des agents réducteurs contenant du bore en présence d'acides en N-(thién-3-yl)méthyl-hydroxylamine, on introduit dans cette dernière le reste acétyle puis, le cas échéant, on transforme le composé d'acide acétohydroxamique de formule I ainsi obtenu, en le dissolvant dans une solution aqueuse de β-cyclodextrine ou d'hydroxypropyl-β-cyclodextrine et en lyophilisant cette solution, en le composé d'inclusion correspondant.

3. Procédé suivant la revendication 2, caractérisé en ce qu'on fait réagir la N-(thién-3-yl)méthylhydroxylamine avec un agent d'acétylation, de préférence avec l'acétanhydride ou le chlorure d'acétyle en présence d'accepteurs d'acides pour produire le composé de formule II 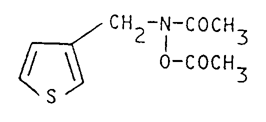 puis on élimine le groupe O-acétyle par addition de bases.

4. Procédé suivant la revendication 2, caractérisé en qu'on réduit l'oxime en N-(thién-3-yl)méthylhydroxylamine avec un borohydrure, notamment le cyanoborohydrure de sodium, en présence d'acides ou avec un complexe borane-amine ou le complexe borane-tétrahydrofuranne en présence d'acides.

5. Médicament, caractérisé en ce qu'il contient comme substance active le composé d'acide acéto-hydroxamique de formule I tel quel ou sous forme d'un composé d'inclusion suivant la revendication 1 et convient pour une administration parentérale, orale, rectale, locale ou intranasale.

6. Médicament suivant la revendication 5, caractérisé en qu'il contient comme substance active, par dose individuelle, 0,01 à 1000 mg de composé d'acide acétohydroxamique de formule I tel quel ou sous forme d'un composé d'inclusion suivant la revendication 1.

7. Médicament suivant l'une des revendications 5 et 6 ou les deux, caractérisé en qu'il convient pour l'administration parentérale et contient comme substance active, par dose unitaire, 0,1 à 1000 mg de composé d'acide acétohydroxamique de formule I tel quel ou sous forme d'un composé d'inclusion suivant la revendication 1.

8. Médicament suivant l'une des revendications 5 et 6 ou les deux, caractérisé en qu'il se présente sous forme de comprimés, de dragées ou de gélules, le cas échéant avec libération retardée de la substance active, et contient comme substance active, par dose unitaire, 0,1 à 1000 mg de composé d'acide acéto-hydroxamique de formule I tel quel ou sous forme d'un composé d'inclusion suivant la revendication 1.

9. Médicament suivant l'une des revendications 5 et 6 ou les deux, sous une forme pulvérisable pour l'administration intranasale ou orale.

10. Procédé de préparation d'un médicament suivant une ou plusieurs des revendications 5 à 9, caractérisé en qu'on met en oeuvre le composé d'acide acéto-hydroxamique de formule I tel quel ou sous forme d'un composé d'inclusion suivant la revendication 1 d'une manière connue avec des excipients, des solvants et/ou des diluants, ainsi que le cas échéant des liants, des agents de désintégration de comprimés et d'autres substances actives, et on prépare une forme dosée unitaire à partir du mélange obtenu.
